# EUROPÄISCHE PATENTSCHRIFT

(11) **EP 3 461 434 B1**
(45) Veröffentlichungstag und Bekanntmachung des Hinweises auf die Patenterteilung: **04.09.2019**
(21) Anmeldenummer: 17193619.8
(22) Anmeldetag: 28.09.2017
(51) Int. Cl.: A61B 17/122, A61B 17/00, A61B 17/12

(54) **CHIRURGISCHER CLIP MIT LAGERHÜLSE**
SURGICAL CLIP WITH BEARING SLEEVE
PINCE CHIRURGICALE COMPRENANT UN MANCHON DE PALIER

(43) Veröffentlichungstag der Anmeldung: 03.04.2019
(73) Patentinhaber: Lazic Besitz GmbH & Co. KG, 78532 Tuttlingen (DE)
(72) Erfinder: LAZIC, Daniel, 78532 Tuttlingen (DE)
(74) Vertreter: Kohler Schmid Möbus Patentanwälte

(56) Entgegenhaltungen:
- EP-A1- 2 752 164
- EP-A1- 2 929 843
- DE-A1- 3 523 031
- DE-A1- 10 309 491

## Beschreibung

Die Erfindung betrifft einen chirurgischen Clip, insbesondere Aneurysma-Clip, mit zwei mittels einer separaten Lagerhülse drehbar miteinander verbundenen Clipteilen und mit einer Schenkelfeder, die die beiden Clipteile in eine Ausgangsdrehstellung vorspannt und deren Windungskörper zumindest teilweise innerhalb der Lagerhülse angeordnet ist, wobei die Lagerhülse unverdrehbar in einer Öffnung des einen, ersten der beiden Clipteile angeordnet und drehbar in einer Öffnung des anderen, zweiten Clipteils gelagert ist und wobei ein erster der beiden Federschenkel der Schenkelfeder an der Lagerhülse und der andere, zweite Federschenkel am zweiten Clipteil abgestützt ist.

Ein derartiger chirurgischer Clip ist beispielsweise durch die EP 2 752 164 A1 bekannt geworden. Dieser bekannte Clip weist eine separate, dünne Lagerhülse aus Metall (z.B. Titan) auf, die unverdrehbar in eine Öffnung des ersten Clipteils eingesteckt ist und auf der das zweite Clipteil mit seiner Öffnung drehbar gelagert ist. Die Lagerhülse bildet somit die Drehlagerung, um die sich das zweite Clipteil dreht. Die Lagerhülse hat einen Steckanschlag in Form eines umlaufenden, dünnen Außenrandes, an den der eine Federschenkel der Schenkelfeder angeschweißt wird, und zwar vorteilhaft bereits vor dem Einstecken der Lagerhülse in das erste Clipteil. Beim Anschweißen des Federschenkels besteht allerdings die Gefahr, dass der dünne Außenrand beschädigt oder gar zerstört wird und die Lagerhülse dann als Ausschussteil aussortiert werden muss, wodurch Fertigungskosten und Montageaufwand deutlich erhöht werden.

Demgegenüber ist es die Aufgabe der vorliegenden Erfindung, bei einem chirurgischen Clip der eingangs genannten Art die Abstützung bzw. Befestigung des einen Federschenkels an der Lagerhülse zu verbessern.

Diese Aufgabe wird erfindungsgemäß dadurch gelöst, dass die Lagerhülse einen - über ihre eine Hülsenstirnseite - axial vorstehenden Vorsprung aufweist, an dem der erste Federschenkel abgestützt ist.

Erfindungsgemäß weist die Lagerhülse also einen Vorsprung auf, an dem sich die Schenkelfeder mit ihrem einen Federschenkel abstützt. Dieser anliegende Federschenkel kann zusätzlich am Vorsprung angeschweißt, ultraschallgeschweißt, angeklebt oder angelötet sein. Im Fall einer Lagerhülse aus Kunststoff kann der anliegende Federschenkel im Vorsprung auch zumindest teilweise formschlüssig eingebettet, insbesondere eingeschmolzen, und dadurch fixiert sein.

Vorzugsweise ist die axiale Höhe des Vorsprungs größer als der Durchmesser des ersten Federschenkels, damit der erste Federschenkel zuverlässig am Vorsprung anliegt. Der Vorsprung kann auch hakenförmig ausgebildet sein, um den ersten Federschenkel darin einhängen und so am Vorsprung axial festlegen zu können.

Besonders bevorzugt ist die radiale Dicke des Vorsprungs größer als die Wandstärke des Lagerabschnitts der Lagerhülse, auf dem das zweite Clipteil drehbar gelagert ist. Dadurch ist der Vorsprung massiver als der Lagerabschnitt der Lagerhülse ausgebildet und kann beschädigungsfrei mit dem anliegenden Federschenkel verschweißt werden.

Ganz besonders bevorzugt steht der Vorsprung an einem umlaufenden Außenrand der Lagerhülse axial vor. Vorteilhaft ist dabei die axiale Höhe des Vorsprungs größer als die Höhe des Außenrands, so dass der anliegende Federschenkel an den Vorsprung angeschweißt werden kann, ohne dabei den Außenrand zu beschädigen. Insbesondere im Fall einer Lagerhülse aus Kunststoff kann der anliegende Federschenkel in den Vorsprung zumindest teilweise eingeschmolzen werden, ohne dass die eigentliche Lagerhülse durch die dabei auftretende Hitze beschädigt wird. Vorzugsweise ist das dem Außenrand gegenüberliegende Hülsenende zu einem Nietkopf umgebogen, so dass die beiden Clipteile auf der Lagerhülse zwischen Außenrand und Nietkopf zusammengehalten werden.

Der zweite Federschenkel kann ebenfalls an dem zweiten Clipteil angeschweißt, angeklebt oder angelötet sein oder aber, was bevorzugt ist, am zweiten Clipteil eingehängt sein oder das zweite Clipteil ähnlich einer Wäscheklammer lediglich umgreifen.

Vorzugsweise ist die Lagerhülse und/oder die Schenkelfeder aus einem Metall, insbesondere Titan, gebildet. Besonders bevorzugt sind die beiden Clipteile aus einem röntgentransparenten Kunststoffmaterial, insbesondere PEEK (Polyetheretherketon), gebildet.

Weitere Vorteile der Erfindung ergeben sich aus der Beschreibung, den Ansprüchen und der Zeichnung. Ebenso können die vorstehend genannten und die noch weiter aufgeführten Merkmale je für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Es zeigen:
- Fign. 1a, 1b: eine erste Ausführungsform des erfindungsgemäßen Clips, der aus zwei Clipteilen und einer Lagerhülse zusammengesteckt ist, in einer geschlossenen Ausgangsdrehstellung in einer Schnittansicht (Fig. 1a) und in einer Draufsicht (Fig. 1b);
- Fign. 2a, 2b: das in Fig. 1 gezeigte eine, erste Clipteil in einer Schnittansicht (Fig. 2a) und in einer Draufsicht auf die Clipteil-Innenseite (Fig. 2b);
- Fign. 3a, 3b: das in Fig. 1 gezeigte andere, zweite Clipteil in einer Schnittansicht (Fig. 3a) und in einer Draufsicht auf die Clipteil-Innenseite (Fig. 3b);
- Fign. 4a, 4b: die in Fig. 1 gezeigte Lagerhülse in einer Schnittansicht (Fig. 4a) und in einer Draufsicht (Fig. 4b);
- Fig. 5: das in Fig. 2 gezeigte erste Clipteil mit der darin unverdrehbar eingesteckten Lagerhülse in einer Schnittansicht analog zu Fig. 2a;
- Fign. 6a, 6b: eine zweite Ausführungsform des erfindungsgemäßen Clips, der aus zwei Clipteilen und einer Lagerhülse zusammengesteckt ist, in einer geschlossenen Ausgangsdrehstellung in einer Schnittansicht (Fig. 6a) und in einer Draufsicht (Fig. 6b);
- Fign. 7a, 7b: das in Fig. 6 gezeigte eine, erste Clipteil in einer Schnittansicht (Fig. 7a) und in einer Draufsicht auf die Clipteil-Innenseite (Fig. 7b);
- Fign. 8a, 8b: das in Fig. 6 gezeigte andere, zweite Clipteil in einer Seitenansicht (Fig. 8a) und in einer Draufsicht auf die Clipteil-Innenseite (Fig. 8b);
- Fign. 9a, 9b: die in Fig. 6 gezeigte Lagerhülse in einer Schnittansicht (Fig. 9a) und in einer Draufsicht (Fig. 9b);
- Fig. 10: das in Fig. 7 gezeigte erste Clipteil mit der darin unverdrehbar eingesteckten Lagerhülse in einer Schnittansicht analog zu Fig. 7a;
- Fign. 11a, 11b: das erste Clipteils einer dritten Ausführungsform des erfindungsgemäßen Clips in einer Schnittansicht (Fig. 11a) und in einer Draufsicht auf die Clipteil-Innenseite (Fig. 11b);
- Fign. 12a, 12b: das zweite Clipteil der dritten Ausführungsform in einer Seitenansicht (Fig. 12a) und in einer Draufsicht auf die Clipteil-Innenseite (Fig. 12b);
- Fign. 13a, 13b: die Lagerhülse der dritten Ausführungsform in einer Schnittansicht (Fig. 13a) und in einer Draufsicht (Fig. 13b); und
- Fig. 14: eine Modifikation eines Vorsprungs des erfindungsgemäßen Clips.

In der folgenden Beschreibung der Zeichnung werden für gleiche bzw. funktionsgleiche Bauteile identische Bezugszeichen verwendet.

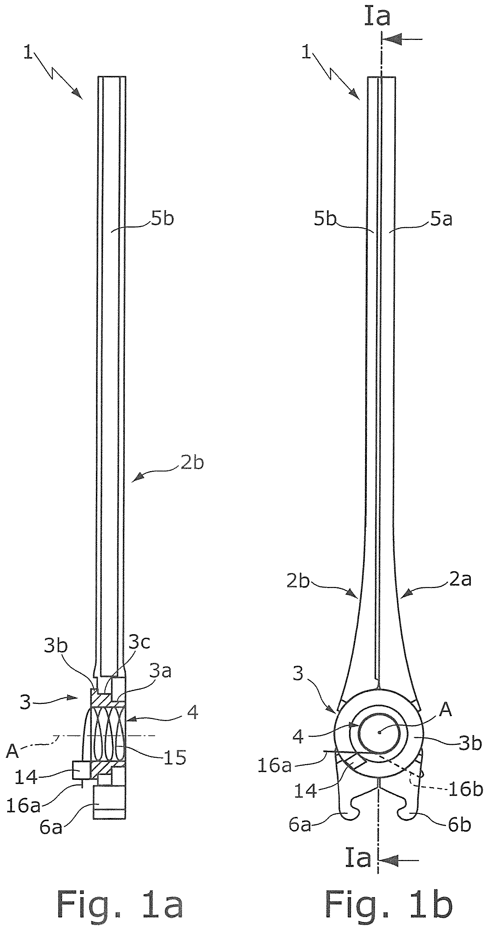

Der in **Fign. 1a, 1b** gezeigte Aneurysma-Clip **1** umfasst zwei doppelarmige Clipteile **2a, 2b,** eine Lagerhülse **3,** die die beiden Clipteilen 2a, 2b drehbar um die Hülsenachse **A** lagert, und eine Schenkelfeder **4,** die die beiden Clipteile 2a, 2b in die gezeigte geschlossene Ausgangsdrehstellung vorspannt.

Die beiden Clipteile 2a, 2b weisen jeweils einen langen Klemmarm **5a, 5b** und einen kurzen Bedienarm **6a, 6b** auf, die bezüglich der Drehachse A einander gegenüberliegen und zueinander parallelversetzt sind. Mithilfe einer zwischen die beiden Bedienarme 6a, 6b greifenden Anlegezange können die Bedienarme 6a, 6b gegen die Schließkraft der Schenkelfeder 4 auseinandergedrückt und dadurch die Klemmarme 5a, 5b geöffnet werden.

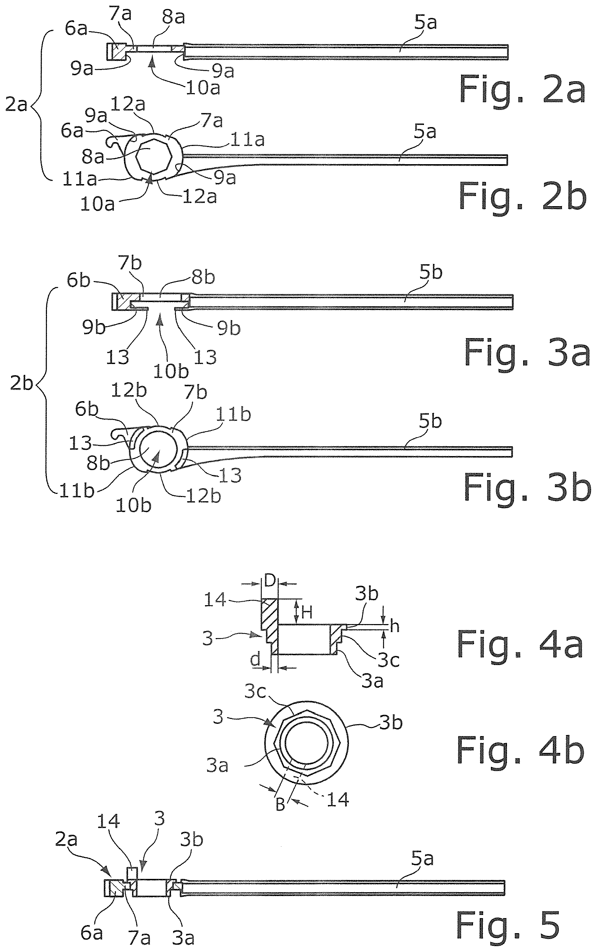

Wie in **Fign. 2a, 2b** gezeigt ist, weist das eine, erste Clipteil 2a zwischen Klemm- und Bedienarm 5a, 6a einen flachen Ringabschnitt (Mittelabschnitt) **7a** mit einer achteckigen Öffnung **8a** auf. Der Ringabschnitt 7a ist gegenüber den Klemm- und Bedienarmen 5a, 6a durch zwei teilzylinderförmige Absätze **9a** axial zurückversetzt, um dadurch eine axial offene Steckaufnahme **10a** auszubilden. Der Ringabschnitt 7a bildet den Boden bzw. die Bodenplatte der Steckaufnahme 10a, und die beiden Absätze 9a bilden zwei bezüglich der Öffnung 8a einander gegenüberliegende Seitenwände der Steckaufnahme 10a. Der Ringabschnitt 7a weist zwei bezüglich der Öffnung 8a einander gegenüberliegende, erste Ringsegmente **11a** und dazwischen jeweils ein zweites Ringsegment **12a** auf, wobei die zweiten Ringsegmente 12a außenseitig gegenüber den ersten Ringsegmenten 11a jeweils radial nach innen zurückversetzt sind.

Wie in **Fign. 3a, 3b** gezeigt ist, weist das andere, zweite Clipteil 2b zwischen Klemm- und Bedienarm 5b, 6b ebenfalls einen flachen Ringabschnitt (Mittelabschnitt) **7b** mit einer kreisrunden Öffnung **8b** auf. Der Ringabschnitt 7b ist gegenüber den Klemm- und Bedienarmen 5b, 6b durch zwei teilzylinderförmige Absätze **9b** axial zurückversetzt, um dadurch eine axial offene Steckaufnahme **10b** auszubilden. Der Ringabschnitt 7b bildet den Boden bzw. die Bodenplatte der Steckaufnahme 10b, und die beiden Absätze 9b bilden zwei bezüglich der Öffnung 8b einander gegenüberliegende Seitenwände der Steckaufnahme 10b. Der Ringabschnitt 7b weist zwei bezüglich der Öffnung 8b einander gegenüberliegende erste Ringsegmente **11b** und dazwischen jeweils ein zweites Ringsegment **12b** auf, wobei die zweiten Ringsegmente 12b außenseitig gegenüber den ersten Ringsegmenten 11b jeweils radial nach innen zurückversetzt sind. Die beiden Absätze 9b sind auf der der Bodenplatte 7b gegenüberliegenden Seite jeweils von einem Überstand **13** übergriffen und dadurch als Umfangsnuten ausgebildet. Mit Ausnahme seiner beiden Überstände 13 kann das zweite Clipteil 2b identisch dem ersten Clipteil 2a ausgebildet sein. Die zweiten Ringsegmente 12b sind zwar funktionsmäßig nicht erforderlich, aber ermöglichen es, dass die beiden Clipteile 2a, 2b aus gleichen Rohteilen gefertigt werden können.

Die in **Fign. 4a, 4b** gezeigte Lagerhülse 3 weist einenends einen Lagerabschnitt **3a** mit kreisrundem Außenquerschnitt, andernends einen umlaufenden Ringbund bzw. Außenrand **3b** sowie dazwischen einen achteckigen Steckabschnitt **3c** auf. Der Steckabschnitt 3c ist gegenüber dem Außenrand 3b und der Lagerabschnitt 3a gegenüber dem Steckabschnitt 3c jeweils radial nach innen zurückversetzt. Weiterhin weist die Lagerhülse 3 einen über den Außenrand 3b axial vorstehenden Vorsprung **14** auf, dessen radiale Dicke **D** größer als die Wandstärke **d** des Lagerabschnitts 3a und dessen axiale Höhe **H** größer als die Höhe **h** des Außenrandes 3b ist. Die Breite **B** des Vorsprungs 14 in Hülsenumfangsrichtung ist ebenfalls jeweils größer als die Wandstärke d des Lagerabschnitts 3a und die Höhe h des Außenrandes 3b.

In **Fig. 5** ist die Lagerhülse 3 mit ihrem achteckigen Steckabschnitt 3c in die achteckige Öffnung 8a des ersten Clipteils 2a eingesteckt und somit darin unverdrehbar gehalten. Die Lagerhülse 3 liegt mit ihrem Außenrand 3b außenseitig am Ringabschnitt 7a des ersten Clipteils 2a an und steht mit ihrem kreisrunden Lagerabschnitt 3a innenseitig über den Ringabschnitt 7a vor. Statt wie gezeigt achteckig, können die Öffnung 8a und der Steckabschnitt 3c auch jeden anderen nicht-kreisrunden Querschnitt aufweisen, z.B. einen anderen polygonen oder einen ovalen Querschnitt.

Zum Montieren des Clips 1 werden das erste Clipteil 2a mit seinen zweiten Ringsegmenten 12a zwischen den beiden Überständen 13 des zweiten Clipteils 2b und das zweite Clipteil 2b mit seinen zweiten Ringsegmenten 12b zwischen den beiden Absätzen 9a des ersten Clipteils 2a ausgerichtet und in dieser geöffneten Montagedrehposition axial ineinander gesteckt, bis sie mit ihren planen Bodenplatten 7a, 7b innenseitig aneinander anliegen und sich ihre Öffnungen 8a, 8b decken. Dabei wird die am ersten Clipteil 2a unverdrehbar eingesteckte Lagerhülse 3 in die Öffnung 8b des zweiten Clipteils 2b eingesteckt, wodurch die beiden Clipteile 2a, 2b drehbar zueinander gelagert sind. Anschließend werden für einen Drehsteckschluss die beiden Clipteile 2a, 2b in Richtung auf ihre geschlossene Endposition gedreht, wodurch die ersten Ringsegmente 11a des ersten Clipteils 2a in die Umfangsnuten 9b des zweiten Clipteils 2b eingreifen und somit die beiden Clipteile 2a, 2b miteinander axial verbunden bzw. entgegen der Steckrichtung verriegelt sind.

Nachdem die Schenkelfeder 4 mit ihrem Federwindungskörper 15 in die Lagerhülse 3 eingesetzt worden ist (Fign. 1a, 1b), liegt der eine, erste Federschenkel 16a der Schenkelfeder 4 in Richtung seiner Federwirkung am Vorsprung 14 der Lagerhülse 3 an und ist vorteilhaft an den Vorsprung 14 angeschweißt. Dazu ist die Höhe H des Vorsprungs 14 bevorzugt größer als der Durchmesser der Federschenkel. Der andere, zweite Federschenkel 16b umgreift - ähnlich einer Wäscheklammer - den Bedienarm 6b des zweiten Clipteils 2b; alternativ kann der zweite Federschenkel 16b aber auch an dem zweiten Clipteil 2b angeschweißt sein. Durch die Schenkelfeder 4 sind einerseits die beiden Clipteile 2a, 2b in ihre geschlossene Ausgangsdrehstellung vorgespannt, und andererseits ist die Lagerhülse 3 unverlierbar im Clip 1 gehalten. Anstatt die Schenkelfeder 4 erst dann in die Lagerhülse 3 einzustecken, wenn diese bereits in das erste Clipteil 2a eingesetzt worden ist, kann auch vor dem Einstecken der Lagerhülse 3 in das erste Clipteil 2a die Schenkelfeder 4 in die Lagerhülse 3 eingesetzt und mit ihrem ersten Federschenkel 16a an den Vorsprung 14 angeschweißt werden.

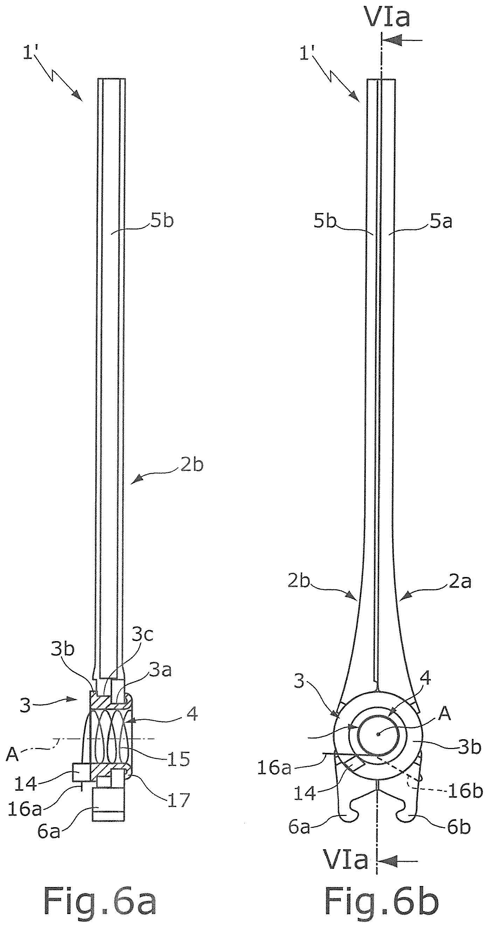

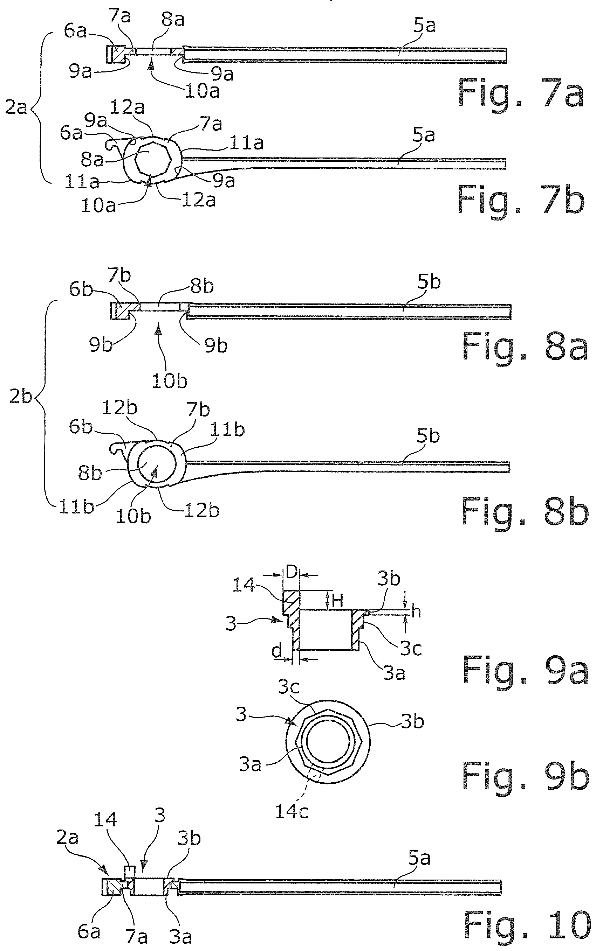

Vom Clip der Fig. 1 unterscheidet sich der in **Fign. 6a, 6b** gezeigte Clip 1 einzig dadurch, dass die beiden Clipteile 2a, 2b nicht durch einen Drehsteckschluss miteinander verbunden sind, sondern durch Umbiegen der Lagerhülse 3. Das in **Fign. 7a, 7b** gezeigte erste Clipteil 2a ist identisch wie das Clipteil 2a der Fign. 2a, 2b und das in **Fign. 8a, 8b** gezeigte zweite Clipteil 2b bis auf die fehlenden Überstände 13 identisch wie das Clipteil 2b der Fign. 3a, 3b ausgebildet. Von der in Fig. 5 gezeigten Lagerhülse unterscheidet sich die in **Fign. 9a, 9b** gezeigte Lagerhülse 3 lediglich durch einen längeren Lagerabschnitt 3a. **Fig. 10** zeigt schließlich das erste Clipteil 2a mit der darin unverdrehbar eingesteckten Lagerhülse 3.

Das erste Clipteil 2a mit der eingesteckten Lagerhülse 3 und das zweite Clipteil 2b werden wie der in Fig. 1 gezeigte Clip 1 montiert und mittels der Schenkelfeder 4 vorgespannt. Die Lagerhülse 3 bildet somit die Drehlagerung, um die sich das zweite Clipteile 2b dreht. Das über die Außenseite des zweiten Clipteils 2b überstehende Ende des längeren Lagerabschnitts 14b der Lagerhülse 3 wird nach außen zu einem Nietkopf **17** umgebogen (Fig. 6a), um die beiden Clipteile 2a, 2b aneinander zu befestigen bzw. axial zusammenzuhalten.

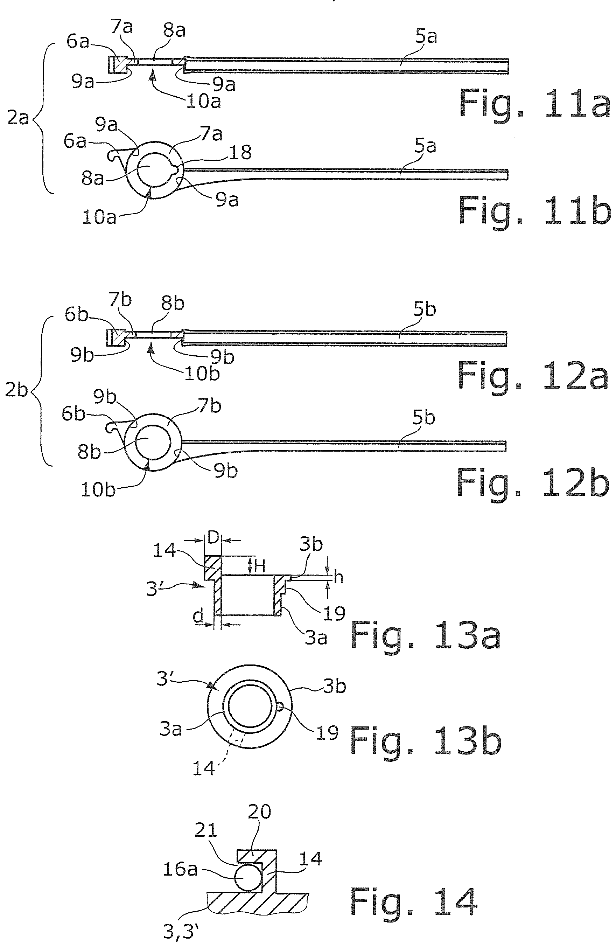

Vom ersten Clipteil der Fig. 7 unterscheidet sich das in **Fign. 11a, 11b** gezeigte erste Clipteil 2a lediglich dadurch, dass die Öffnung 8a kreisrund ausgebildet ist und außen eine zusätzliche radiale Aussparung **18** aufweist. Das in **Fign. 12a, 12b** gezeigte zweite Clipteil 2b ist mit Ausnahme der fehlenden Aussparung baugleich zum ersten Clipteil 2a ausgebildet. Von der Lagerhülse 3 der Fig. 9 unterscheidet sich die in Fign. **13a, 13b** gezeigte Lagerhülse 3' lediglich dadurch, dass hier kein achteckiger Steckabschnitt, sondern stattdessen außen am Lagerabschnitt 3a ein Vorsprung **19** vorhanden ist. Die in das erste Clipteil 2a eingesteckte Lagehülse 3' greift mit ihrem Vorsprung 19 in die Aussparung 19 der Öffnung 8a ein und ist somit in der Öffnung 8a unverdrehbar gehalten. Das erste Clipteil 2a mit der eingesteckten Lagerhülse 3' und das zweite Clipteil 2b werden wie der in Fig. 6 gezeigte Clip 1 montiert und mittels der Schenkelfeder 4 vorgespannt. Die Lagerhülse 3 bildet somit die Drehlagerung, um die sich das zweite Clipteile 2b dreht, und hält die beiden Clipteile 2a, 2b zwischen ihrem Außenrand 3a und ihrem Nietkopf 17 axial zusammen.

Wie in **Fig. 14** gezeigt, kann der Vorsprung 14 der Lagerhülse 3, 3' hakenförmig mit einem Überstand **20** und somit mit einer Nut **19** ausgebildet sein, in die der erste Federschenkel 16a eingelegt und, weil axial übergriffen, axial festgelegt ist.

## Patentansprüche

1. Chirurgischer Clip (1; 1'), insbesondere Aneurysma-Clip, mit zwei mittels einer separaten Lagerhülse (3; 3') drehbar miteinander verbundenen Clipteilen (2a, 2b) und mit einer Schenkelfeder (4), die die beiden Clipteile (2a, 2b) in eine Ausgangsdrehstellung vorspannt und deren Windungskörper (15) zumindest teilweise innerhalb der Lagerhülse (3; 3') angeordnet ist, wobei die Lagerhülse (3; 3') unverdrehbar in einer Öffnung (8a) des einen, ersten (2a) der beiden Clipteile angeordnet und drehbar in einer Öffnung (8b) des anderen, zweiten Clipteils (2b) gelagert ist und wobei ein erster (16a) der beiden Federschenkel der Schenkelfeder (4) an einem axial in Richtung der Drehachse (A) der beiden Clipteile (2a, 2b) vorstehenden Vorsprung (14) der Lagerhülse (3; 3') und der andere, zweite Federschenkel (16b) am zweiten Clipteil (2b) abgestützt ist,
**dadurch gekennzeichnet,**
**dass** der erste Federschenkel (16a) in Richtung seiner Federwirkung außen an dem Vorsprung (14) anliegt.

2. Chirurgischer Clip nach Anspruch 1, **dadurch gekennzeichnet, dass** der erste Federschenkel (16a) am Vorsprung (14) angeschweißt, angeklebt oder angelötet ist.

3. Chirurgischer Clip nach Anspruch 1, **dadurch gekennzeichnet, dass** der erste Federschenkel (16a) im Vorsprung (14) zumindest teilweise formschlüssig eingebettet, insbesondere eingeschmolzen ist.

4. Chirurgischer Clip nach einem der vorhergehenden Ansprüche, **dadurch gekennzeichnet, dass** die axiale Höhe (H) des Vorsprungs (14) größer als der Durchmesser des ersten Federschenkels (16a) ist.

5. Chirurgischer Clip nach einem der vorhergehenden Ansprüche, **dadurch gekennzeichnet, dass** der Vorsprung (14) hakenförmig ausgebildet ist und den ersten Federschenkel (16a) radial übergreift.

6. Chirurgischer Clip nach einem der vorhergehenden Ansprüche, **dadurch gekennzeichnet, dass** die radiale Dicke (D) des Vorsprungs (14) größer als die Wandstärke (d) des Lagerabschnitts (3a) ist, auf dem das zweite Clipteil (2b) drehbar gelagert ist.

7. Chirurgischer Clip nach einem der vorhergehenden Ansprüche, **dadurch gekennzeichnet, dass** der Vorsprung (14) an einem umlaufenden Ringbund (3b) der Lagerhülse (3; 3'), der in radialer Richtung an einem Ende der Lagerhülse (3; 3') absteht und zur axialen Abstützung der Lagerhülse (3; 3') am ersten Clipteil (2a) dient, axial vorsteht.

8. Chirurgischer Clip nach Anspruch 7, **dadurch gekennzeichnet, dass** die axiale Höhe (H) des Vorsprungs (14) größer als die Höhe (h) des Ringbunds (3b) ist.

9. Chirurgischer Clip nach einem der vorhergehenden Ansprüche, **dadurch gekennzeichnet, dass** das dem Vorsprung (14) gegenüberliegende Hülsenende (3a) der Lagerhülse (3; 3') zu einem Nietkopf (17) umgebogen ist.

10. Chirurgischer Clip nach einem der vorhergehenden Ansprüche, **dadurch gekennzeichnet, dass** die Lagerhülse (3; 3') einen Steckabschnitt (3c; 19) mit nicht-kreisförmiger Außenkontur aufweist, der in die nicht-kreisförmig ausgebildete Öffnung (8a) des ersten Clipteils (2a) unverdrehbar eingesteckt ist.

11. Chirurgischer Clip nach einem der vorhergehenden Ansprüche, **dadurch gekennzeichnet, dass** die beiden Clipteile (2a, 2b) jeweils einen Klemmarm (5a, 5b), einen Bedienarm (6a, 6b) und einen dazwischen befindlichen Mittelabschnitt (7a, 7b) mit der Öffnung (8a, 8b) aufweisen.

12. Chirurgischer Clip nach einem der vorhergehenden Ansprüche, **dadurch gekennzeichnet, dass** der zweite Federschenkel (16b) der Schenkelfeder (4) an dem zweiten Clipteil (2b) eingehängt ist.

13. Chirurgischer Clip nach einem der vorhergehenden Ansprüche, **dadurch gekennzeichnet, dass** der zweite Federschenkel (16b) der Schenkelfeder (4) an dem zweiten Clipteil (2b) angeschweißt, angeklebt oder angelötet ist.

14. Chirurgischer Clip nach einem der vorhergehenden Ansprüche, **dadurch gekennzeichnet, dass** die Lagerhülse (3; 3') und/oder die Schenkelfeder (4) aus einem Metall, insbesondere Titan, gebildet ist.

15. Chirurgischer Clip nach einem der vorhergehenden Ansprüche, **dadurch gekennzeichnet, dass** die beiden Clipteile (2a, 2b) aus einem Kunststoff, insbesondere PEEK, gebildet sind.

## Claims

1. Surgical clip (1; 1'), in particular an aneurysm clip, comprising two clip parts (2a, 2b) that are rotatably interconnected by means of a separate bearing sleeve (3, 3'), and a leg spring (4) which pretensions the two clip parts (2a, 2b) to an initial rotary position, the coil body (15) of said leg spring (4) at least partially being disposed within the bearing sleeve (3, 3'), wherein the bearing sleeve (3, 3') is disposed so as to be non-rotatable in an opening (8a) of the first (2a) of the two clip parts and is disposed so as to be rotatable in an opening (8b) of the other, second clip part (2b), and wherein a first (16a) of the two spring legs of the leg spring (4) is supported on a protrusion (14) of the bearing sleeve (3; 3'), which axially protrudes in the direction of the axis of rotation (A) of the two clip parts (2a, 2b), and the other, second spring leg (16b) is supported on the second clip part (2b),
**characterized in that**
the first spring leg (16a) abuts the outside of the protrusion (14) in the direction of its spring action.

2. Surgical clip according to claim 1, **characterized in that** the first spring leg (16a) is welded, adhesively bonded, or soldered to the protrusion (14).

3. Surgical clip according to claim 1, **characterized in that** the first spring leg (16a) is at least partially embedded in a form-fitting manner, in particular melted, in the protrusion (14).

4. Surgical clip according to one of the preceding claims, **characterized in that** the axial height (H) of the protrusion (14) is larger than the diameter of the first spring leg (16a).

5. Surgical clip according to one of the preceding claims, **characterized in that** the protrusion (14) is configured so as to be hook-shaped and engages radially across the first spring leg (16a).

6. Surgical clip according to one of the preceding claims, **characterized in that** the radial thickness (D) of the protrusion (14) is larger than the wall thickness (d) of the bearing portion (3a) on which the second clip part (2b) is rotatably mounted.

7. Surgical clip according to one of the preceding claims, **characterized in that** the protrusion (14) protrudes axially on an encircling annular collar (3b) of the bearing sleeve (3; 3'), which protrudes radially at one end of the bearing sleeve (3; 3') and is used for axial support of the bearing sleeve (3; 3') on the first clip part (2a).

8. Surgical clip according to claim 7, **characterized in that** the axial height (H) of the protrusion (14) is larger than the height (h) of the annular collar (3b).

9. Surgical clip according to one of the preceding claims, **characterized in that** the sleeve end (3a) of the bearing sleeve (3; 3') facing the protrusion (14) is bent back so as to form a rivet head (17).

10. Surgical clip according to one of the preceding claims, **characterized in that** the bearing sleeve (3; 3') has a plug-in portion (3c; 19) which has a non-circular outer contour and which is plug-fitted so as to be non-rotatable **in that** opening (8a) of the first clip part (2a) that is configured so as to be non-circular.

11. Surgical clip according to one of the preceding claims, **characterized in that** the two clip parts (2a, 2b) have each one clamping arm (5a, 5b), one operating arm (6a, 6b), and one middle portion (7a, 7b) located therebetween, said middle portion (7a, 7b) having the opening (8a, 8b).

12. Surgical clip according to one of the preceding claims, **characterized in that** the second spring leg (16b) of the leg spring (4) is hooked onto the second clip part (2b).

13. Surgical clip according to one of the preceding claims, **characterized in that** the second spring leg (16b) of the leg spring (4) is welded, adhesively bonded, or soldered to the second clip part (2b).

14. Surgical clip according to one of the preceding claims, **characterized in that** the bearing sleeve (3; 3') and/or the leg spring (4) are/is formed from a metal, in particular titanium.

15. Surgical clip according to one of the preceding claims, **characterized in that** the two clip parts (2a, 2b) are formed from a plastics material, in particular PEEK.

## Revendications

1. Pince chirurgicale (1 ; 1'), notamment pince de traitement des anévrismes, comprenant deux parties de pincement (2a, 2b) reliées l'une à l'autre avec faculté de rotation, au moyen d'un coussinet de support (3 ; 3') distinct, et un ressort (4) à branches qui précontraint les deux parties de pincement (2a, 2b) vers une position initiale prise par rotation, et dont le corps d'enroulement (15) est au moins partiellement logé à l'intérieur dudit coussinet de support (3 ; 3'), sachant que ledit coussinet de support (3 ; 3') est disposé, sans faculté de rotation, dans un orifice (8a) de l'une des deux parties de pincement matérialisant la première partie (2a), et est monté à rotation dans un orifice (8b) de l'autre partie de pincement matérialisant la seconde partie (2b), et sachant qu'une première branche (16a), au sein des deux branches dudit ressort (4) à branches, est en appui contre une saillie (14) dudit coussinet de support (3 ; 3') qui dépasse axialement en direction de l'axe de rotation (A) des deux parties de pincement (2a, 2b), et que l'autre branche, ou seconde branche (16b) dudit ressort est en appui contre ladite seconde partie de pincement (2b),
**caractérisée par le fait**
**que** la première branche (16a) du ressort est extérieurement en applique contre la saillie (14), dans la direction de son action élastique.

2. Pince chirurgicale selon la revendication 1, **caractérisée par le fait que** la première branche (16a) du ressort est rapportée sur la saillie (14) par soudage, par collage ou par brasage.

3. Pince chirurgicale selon la revendication 1, **caractérisée par le fait que** la première branche (16a) du ressort est au moins partiellement noyée dans la saillie (14) par complémentarité de formes, en particulier intégrée par fusion.

4. Pince chirurgicale selon l'une des revendications précédentes, **caractérisée par le fait que** la hauteur axiale (H) de la saillie (14) est supérieure au diamètre de la première branche (16a) du ressort.

5. Pince chirurgicale selon l'une des revendications précédentes, **caractérisée par le fait que** la saillie (14) est réalisée en forme de crochet, et coiffe la première branche (16a) du ressort dans le sens radial.

6. Pince chirurgicale selon l'une des revendications précédentes, **caractérisée par le fait que** l'épaisseur radiale (D) de la saillie (14) est supérieure à l'épaisseur de paroi (d) de la zone de montage (3a) sur laquelle la seconde partie de pincement (2b) est montée à rotation.

7. Pince chirurgicale selon l'une des revendications précédentes, **caractérisée par le fait que** la saillie (14) dépasse, dans le sens axial, au-delà d'un collet annulaire circonférentiel (3b) du coussinet de support (3 ; 3') qui est en débord, dans le sens radial, au niveau d'une extrémité dudit coussinet de support (3 ; 3'), et sert à l'appui axial dudit coussinet de support (3 ; 3') contre la première partie de pincement (2a).

8. Pince chirurgicale selon la revendication 7, **caractérisée par le fait que** la hauteur axiale (H) de la saillie (14) est supérieure à la hauteur (h) du collet annulaire (3b).

9. Pince chirurgicale selon l'une des revendications précédentes, **caractérisée par le fait que** l'extrémité (3a) du coussinet de support (3 ; 3'), qui pointe à l'opposé de la saillie (14), est recourbée en une tête de rivet (17).

10. Pince chirurgicale selon l'une des revendications précédentes, **caractérisée par le fait que** le coussinet de support (3 ; 3') est pourvu d'une zone d'emboîtement (3c ; 19) à profil extérieur non circulaire qui est emboîtée, sans faculté de rotation, dans l'orifice non circulaire (8a) de la première partie de pincement (2a).

11. Pince chirurgicale selon l'une des revendications précédentes, **caractérisée par le fait que** les deux parties de pincement (2a, 2b) sont respectivement dotées d'un bras de coincement (5a, 5b), d'un bras d'actionnement (6a, 6b) et d'une zone médiane intercalaire (7a, 7b) percée de l'orifice (8a, 8b).

12. Pince chirurgicale selon l'une des revendications précédentes, **caractérisée par le fait que** la seconde branche (16b) du ressort (4) à branches est accrochée à la seconde partie de pincement (2b).

13. Pince chirurgicale selon l'une des revendications précédentes, **caractérisée par le fait que** la seconde branche (16b) du ressort (4) à branches est rapportée sur la seconde partie de pincement (2b) par soudage, par collage ou par brasage.

14. Pince chirurgicale selon l'une des revendications précédentes, **caractérisée par le fait que** le coussinet de support (3 ; 3'), et/ou le ressort (4) à branches, consiste(nt) en un métal et notamment en du titane.

15. Pince chirurgicale selon l'une des revendications précédentes, **caractérisée par le fait que** les deux parties de pincement (2a, 2b) consistent en une matière plastique, notamment en du PEEK.
